# EUROPEAN PATENT APPLICATION

(11) **EP 3 992 992 A1**
(43) Date of publication of application: **04.05.2022**
(21) Application number: 21199299.5
(22) Date of filing: 28.09.2021
(51) Int. Cl.: H01F 1/00

(54) **MULTI-COMPONENT MESOCRYSTALLINE NANOPARTICLES AND METHOD OF MANUFACTURING THE SAME**

(30) Priority: 30.10.2020 KR 20200143429
(71) Applicant: Korea University Research and Business Foundation, Seoul 02841 (KR)
(72) Inventor: KIM, Young Keun, 06009 Seoul (KR); KO, Min Jun, 07974 Seoul (KR); BYUN, Sang Won, 02842 Seoul (KR); PARK, Bum Chul, 04700 Seoul (KR); KOO, Thomas Myeongseok, 04700 Seoul (KR)
(74) Representative: Eisenführ Speiser

(57) **Abstract**

A multi-component mesocrystalline nanoparticle is provided. The multi-component mesocrystalline nanoparticle includes an iron oxide nanocluster; and metal oxide nanocrystals bound to a surface of the iron oxide.

## Description

### CROSS-REFERENCE TO RELATED APPLICATION

This application claims priority to and the benefit of Korean Patent Application No. 2020-0143429, filed October 30, 2020, the disclosure of which is incorporated herein by reference in its entirety.

### BACKGROUND

### 1. Field of the Invention

The present invention relates to a multi-component mesocrystalline nanoparticle, and more particularly, to a multi-component mesocrystalline nanoparticle including an iron oxide nanocluster; and metal oxide nanocrystals bound to an iron oxide surface of the iron oxide nanocluster.

### 2. Discussion of Related Art

Nanoparticles having magnetic properties have come into the spotlight in nanomedical fields such as biosensors for diagnosing diseases, hyperthermia therapy, drug delivery systems, contrast media for magnetic resonance imaging, and the like. In the above-described application fields, various types of research on the regulation of properties of the magnetic nanoparticles have been conducted to achieve high efficiency. Such magnetic nanoparticles are synthesized using various synthesis processes such as a high-temperature thermal decomposition method, a coprecipitation method, a sol-gel method, an electrochemical method, a sound synthesis method, a high-temperature spraying method, and the like. To apply the nanoparticles to the above-described application fields, each of the nanoparticles should have uniform physical properties such as uniform shapes. Therefore, solvothermal synthesis methods such as a high-temperature thermal decomposition method, a sol-gel method, and the like have been mainly used to manufacture nanoparticles having a uniform shape.

With the development of nanotechnology, a lot of research on nano-material technology, which imparts not only magnetic properties but also other functionalities such as plasmonic, light emission, ferroelectric, and catalytic properties, and the like to the nanoparticles to overcome the limitations of the existing mono-functional materials, technology for manufacturing the nano-materials, and technology for applying the nano-materials has been conducted. Also, research on the complexation of other materials into forms such as a core-shell structure, a Janus structure, a dimeric structure, a multi-component mesocrystalline nanoparticle cluster structure, and the like has been conducted to impart multi-functionalities to one nanoparticle.

Among the above-described various structures, the multi-component mesocrystalline nanoparticle cluster structure has advantages in that it may make use of the characteristics expressed by each building block material, and the characteristics which depend on the size of an individual building block, and may also simultaneously adjust the aggregate characteristics expressed from an interaction (e.g., arrangement) between the building blocks.

In general, additional preparatory processes such as a process of preparing each of two nanoparticles used as building blocks, a process of removing a metal ion precursor and a surfactant present on surfaces of the nanoparticles, and a process of replacing a reaction solvent are required to form nanocomposites having a cluster structure. Thereafter, a complexation process using a self-assembled polymer is also required (Patent Document 1).

Therefore, there is a need for a novel method of manufacturing multi-component mesocrystalline nanoparticles having multi-functional properties.

### [Related-Art Document]

### [Patent Document]

Patent Document 1: Korean Patent Publication No. 10-2011-0006624

### SUMMARY OF THE INVENTION

The present invention is directed to providing a multi-component mesocrystalline nanoparticle manufactured using a simple and stable method.

One aspect of the present invention provides a multi-component mesocrystalline nanoparticle which includes:
an iron oxide nanocluster; and
metal oxide nanocrystals bound to an iron oxide surface of the iron oxide nanocluster,
wherein the metal oxide nanocrystals are bound to acrylate groups formed on the iron oxide surface.

Another aspect of the present invention provides a method of manufacturing the above-described multi-component mesocrystalline nanoparticles, which includes:
allowing a mixture including an iron ion precursor, an anionic ligand, and a solvent to react at 100 to 300°C; and
injecting a metal ion precursor solution into the reaction product of the previous step to react with the reaction product,
wherein the anionic ligand includes an acetate-based compound and an acrylate-based compound.

Still another aspect of the present invention provides a method of purifying contaminated water, which includes:
allowing the above-described multi-component mesocrystalline nanoparticles and contaminated water to react under ultraviolet or visible light to decompose contaminants in the wastewater; and
recovering the multi-component mesocrystalline nanoparticles using a magnet.

Yet another aspect of the present invention provides a method of detecting or imaging an analyte, which includes:
functionalizing biomolecules capable of binding to an analyte to be detected on surfaces of the above-described multi-component mesocrystalline nanoparticles;
exposing the functionalized multi-component mesocrystalline nanoparticles to a sample including one or more analytes; and
identifying the analytes bound to the multi-component mesocrystalline nanoparticles.

### BRIEF DESCRIPTION OF THE DRAWINGS

The above and other objects, features and advantages of the present invention will become more apparent to those of ordinary skill in the art by describing in detail exemplary embodiments thereof with reference to the attached drawings, in which:
FIG. 1 is a schematic diagram showing a synthesis mechanism of multi-component mesocrystalline nanoparticles according to the present invention;
FIG. 2 shows transmission electron microscope images and energy dispersive spectrometry (EDS) images of the multi-component mesocrystalline nanoparticles manufactured by the manufacturing method in Example 1-1;
FIG. 3 shows transmission electron microscope (TEM) images (a and b) and energy dispersive spectrometry (EDS) images (c and d) of the multi-component nanoparticles manufactured by the manufacturing method in Comparative Example 1-1;
FIG. 4 shows the X-ray diffraction pattern results and selected area electron diffraction (SAED) pattern images of the multi-component mesocrystalline nanoparticles manufactured in Example 1-1;
FIG. 5 shows the results of analyzing microstructures of the multi-component mesocrystalline nanoparticles manufactured by the manufacturing method in Example 1-1 using a Raman spectrometer;
FIG. 6 shows transmission electron microscope images and energy dispersive spectrometry images of the multi-component mesocrystalline nanoparticles;
FIG. 7 shows the results of analyzing the surface state of the multi-component mesocrystalline nanoparticles at the time point of injection of a zinc ion precursor after addition of sodium acrylate;
FIG. 8 shows transmission electron microscope images of the multi-component mesocrystalline nanoparticles;
FIG. 9 shows transmission electron microscope images and selected area electron diffraction pattern images of the multi-component mesocrystalline nanoparticles according to the content of the zinc ion precursor;
FIG. 10 shows the X-ray photoelectron spectroscopy results of the multi-component mesocrystalline nanoparticles according to the content of the zinc ion precursor;
FIG. 11 shows transmission electron microscope (TEM) images, energy dispersive spectrometry (EDS) images, and the X-ray photoelectron spectroscopy results of the multi-component mesocrystalline nanoparticles manufactured by the manufacturing method in Example 2-1;
FIG. 12 shows transmission electron microscope (TEM) images, energy dispersive spectrometry (EDS) images, and the X-ray photoelectron spectroscopy results of the multi-component mesocrystalline nanoparticles manufactured by the manufacturing method in Example 3-1;
FIG. 13 shows the photocatalytic properties using the multi-component mesocrystalline nanoparticles manufactured by the method in Example 1-1; and
FIG. 14 shows a schematic diagram and a scanning electron microscope (SEM) image of the multi-component nanoparticles with a spike-like shape synthesized by a hydrothermal synthesis method in Example 1-2.

### DETAILED DESCRIPTION OF EXEMPLARY EMBODIMENTS

Hereinafter, exemplary embodiments of the present invention will be described in detail. However, the present invention is not limited to the embodiments disclosed below, but can be implemented in various forms. The following embodiments are described in order to enable those of ordinary skill in the art to embody and practice the present invention.

The present invention relates to a multi-component mesocrystalline nanoparticle, which includes:
an iron oxide nanocluster; and
metal oxide nanocrystals bound to an iron oxide surface of the iron oxide nanocluster,
wherein the metal oxide nanocrystals are bound to acrylate groups formed on the iron oxide surface.

In the present invention, the term "mesocrystal" refers to a structure in which small unit crystals aggregate with each other, and may exhibit new aggregate characteristics which are not expressed in the individual unit crystals. In the present invention, the "multi-component mesocrystalline nanoparticles" may form a mesocrystalline structure with an iron oxide and a metal oxide.

Hereinafter, the present invention will be described in detail.

In the present invention, the term "nanocluster" is a term commonly used in the art, and refers to an aggregate formed by aggregation of nanoparticles. In this case, the nanoparticles may also form a mesocrystalline structure.

That is, in the present invention, the iron oxide nanocluster refers to an aggregate formed by aggregation of iron oxide nanoparticles.

According to one embodiment, iron oxide nanoparticles (which may also be referred to as iron oxide) have the same dictionary meaning as a compound of iron and oxygen, and may also include other metals such as cobalt, nickel, manganese, and the like in the structure thereof. Types of such an iron oxide are not particularly limited, and may include, for example, one or more selected from the group consisting of Fe₂O₃, Fe₃O₄, CoFe₂O₄, NiFe₂O₄, ZnFe₂O₄, MgFe₂O₄, and MnFe₂O₄. In the present invention, a magnetic property may be imparted to the multi-component mesocrystalline nanoparticles through the iron oxide cluster.

According to one embodiment, the iron oxide nanocluster may be spherical. In the present invention, the term "spherical" may encompass all types of shapes having a round appearance as well as spheres having a mathematical definition as three-dimensional shapes in which all points are equidistant from one point.

According to one embodiment, the average particle size of the iron oxide nanoparticles constituting the iron oxide nanoclusters is not limited, but may be, for example, in a range of 1 to 20 nm. Also, the iron oxide nanoclusters may have an average particle size of 10 to 500 nm. When the size of the iron oxide nanoclusters is too small, the iron oxide nanoclusters may not be applied for use for magnetic detection purposes, and the like due to a small amount of magnetic moment. Also, when the particle size of the iron oxide nanoparticles is greater than 20 nm, a phenomenon in which the iron oxide nanoclusters are converted into a ferromagnetic substance such that iron oxide particles stick together occurs. Therefore, it is desirable to set the size of the iron oxide nanoparticles and the iron oxide clusters within the above range.

In the present invention, metal oxide nanocrystals are bound to surfaces of the iron oxide nanoparticles constituting the iron oxide nanoclusters. The metal oxide nanocrystals may impart photocatalytic and antioxidant properties to the multi-component mesocrystalline nanoparticles.

According to one embodiment, types of the metal oxide are not particularly limited, and may, for example, include one or more selected from the group consisting of zinc oxide (ZnO), cerium oxide (CeO₂), manganese oxide (MnO₂), nickel oxide (NiO, Ni₂O₃), cobalt oxide (Co₃O₄, CoO), magnesium oxide (MgO), zinc ferrite (ZnFe₂O₄), cerium ferrite (CeFe₂O₄), manganese ferrite (MnFe₂O₄), nickel ferrite (NiFe₂O₄), cobalt ferrite (CoFe₂O₄), magnesium ferrite (MgFe₂O₄), and cerium-doped iron oxide (CeₓFe₃₋ₓO₄).

According to one embodiment, the metal oxide nanocrystal may also be spherical, or may be spike-shaped or acicular. The spike shape may refer to a cylindrical crystal form. In this case, the cylindrical form may be a single cylinder or a polygonal prism. Also, the acicular shape may refer to a crystal form whose end tapers in the spike shape.

According to one embodiment, when the metal oxide nanocrystals are spherical, the average particle size of the metal oxide nanocrystals may be in a range of 1 to 100 nm, or a range of 5 to 50 nm. Also, when the metal oxide nanocrystals are spike-shaped or acicular, the average height of the metal oxide nanocrystals may be in a range of 100 to 2,000 nm. When the metal oxide nanocrystals are spike-shaped, the thickness of the metal oxide nanocrystals may be in a range of 10 to 150 nm.

Also, the present invention relates to a method of manufacturing the above-described multi-component mesocrystalline nanoparticles.

The multi-component mesocrystalline nanoparticles according to the present invention may be manufactured by the following steps:
(S1) allowing a mixture including an iron ion precursor, an anionic ligand, and a solvent to react at 100 to 300°C; and
(S2) injecting a metal ion precursor solution into the reaction product of the previous step to react with the reaction product

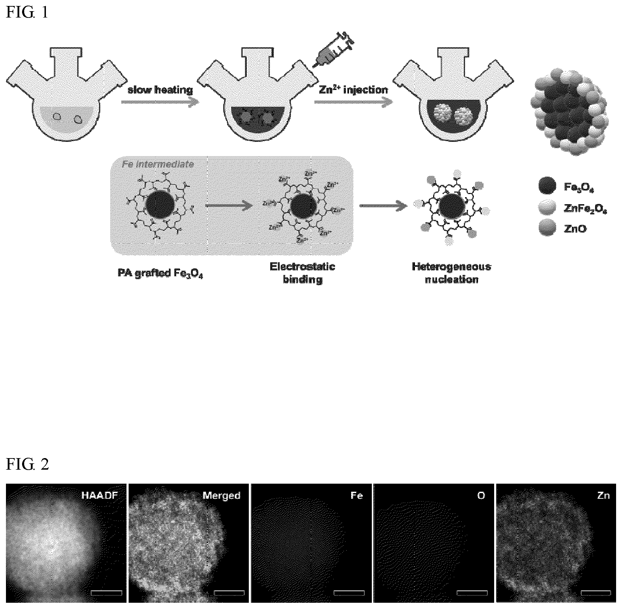

In the present invention, FIG. 1 shows a synthesis mechanism of the multi-component mesocrystalline nanoparticles when the metal ion precursor is a zinc ion precursor. As shown in FIG. 1, in a synthesis process of the multi-component mesocrystalline nanoparticles, polyacrylates are formed on surfaces of iron oxide nanoparticles, and bind to zinc ions due to electrostatic attraction. Thereafter, zinc oxide crystals may be formed through heterogeneous nucleation and growth processes.

The steps (S1) and (S2) of the present invention may be performed in a single-stage reactor. Therefore, the multi-component mesocrystalline nanoparticles may be stably manufactured using an easy and simple process. Also, the manufacturing method of the present invention may manufacture multi-component mesocrystalline nanoparticles without any solvent replacement, such as diethylene glycol, which has a high reducing power, and may also manufacture multi-component mesocrystalline nanoparticles having a reduced crystal grain size of the iron oxide nanoparticles and a wide specific surface area.

In the present invention, the step (S1) is a step of allowing a mixture including an iron ion precursor, an anionic ligand, and a solvent to react at 100 to 300°C. According to an embodiment of the present invention, surfaces of the iron oxide nanoparticles are uniformly coated with sodium acrylate which is one anionic ligand.

According to one embodiment, types of the iron ion precursor are not particularly limited, and may be, for example, iron chloride hexahydrate (FeCl₃·6H₂O). In the present invention, one or more selected from the group consisting of zinc chloride (ZnCl₂), magnesium chloride hexahydrate (MgCl₂·6H₂O), manganese chloride tetrahydrate (MnCl₂·4H₂O), nickel chloride hexahydrate (NiCl₂·6H₂O), and cobalt chloride hexahydrate (CoCl₂·6H₂O) may also be further used together with the iron ion precursor.

According to one embodiment, types of the anionic ligand are not particularly limited, and an acetate-based compound and an acrylate-based compound may be used. The acetate-based compound may include one or more selected from the group consisting of sodium acetate, potassium acetate, and ammonium acetate, and the acrylate-based compound may include one or more selected from the group consisting of sodium acrylate, poly(acrylic acid), and poly(acrylic acid sodium salt).

The acetate-based compound and the acrylate-based compound may be included at a content ratio of 10,000:1 to 1:1.

Also, the anionic ligand and the iron ion precursor may be included at a content ratio of 100:1 to 1:100.

According to one embodiment, the solvent may serve as a reducing agent as well as the solvent. Types of such a solvent are not particularly limited, and may be, for example, ethylene glycol. Particularly, the solvent may be ethylene glycol, diethylene glycol, triethylene glycol, or tetraethylene glycol.

A content of the solvent may be in a range of 1:1 to 1:100,000 (solvent: iron ion precursor) relative to the iron ion precursor.

In this step, the iron ion precursor and the anionic ligand are dissolved and hydrolyzed in the solvent, and an amorphous gel is formed by a condensation reaction between the hydrolyzed iron ion precursors. In such a process, when iron chloride hexahydrate is used as the iron ion precursor, a temperature range in which the corresponding reaction occurs is in a range of 60 to 80°C, the temperature of which overlaps a temperature zone at which sodium acrylate polymerizes into polyacrylate. Therefore, a reaction temperature may be sufficiently slowly raised to coat (form) the polyacrylate onto a surface of crystalline lepidocrocite. The lepidocrocite is phase-transitioned into a crystalline metal oxide at a high temperature to form a metal oxide. Also, the formed metal oxide may be reduced, and aggregated into a crystalline magnetic substance.

According to one embodiment, this step may be performed at 100 to 300°C, or at 180 to 250°C. Particularly, this step may be performed by raising the temperature from room temperature (R.T) to the reaction temperature at 0.1 to 20°C/minute. Also, the reaction time may be in a range of 1 to 24 hours. The iron oxide nanoclusters in which acrylate groups are formed on surfaces of the iron oxide nanoparticles may be manufactured under the above reaction conditions.

In the present invention, the step (S2) is a step of injecting a metal ion precursor solution into the reaction product of the step (S1) to react with the reaction product.

In this step, the metal oxide nanocrystals may be formed on a surface of the iron oxide. In the step (S1), because the acrylate-based compound is used as the anionic ligand, polyacrylate groups are formed on surfaces of the manufactured iron oxide nanoparticles. Therefore, metal ions bind to the polyacrylate groups via electrostatic attraction in this step. Thereafter, nanocrystals may be synthesized through heterogeneous nucleation and growth processes.

According to one embodiment, the metal ion precursor in the metal ion precursor solution may include one or more selected from the group consisting of zinc acetate dihydrate (Zn(CH₃COO)₂·2H₂O), manganese acetate dihydrate (Mn(CH₃COO)₂·2H₂O), cerium acetate (Ce(CH₃COO)·xH₂O), magnesium acetate tetrahydrate (Mg(CH₃COO)₂·4H₂O), cobalt acetate tetrahydrate (Co(CH₃COO)₂·4H₂O), and nickel acetate tetrahydrate (Ni(CH₃COO)₂·4H₂O). In this case, the solvent described above in the step (S1) may be used as the solvent in the solution. Also, the same solvent may be used.

A content of the metal ion precursor may be in a range of 0.01 mmol to 1 mol. Also, a molar ratio of iron ions of the iron ion precursor and metal ions of the metal ion precursor may be in a range of 1:0.01 to 1:1, or in a range of 1:0.1 to 1:0.2.

According to one embodiment, the reaction may be performed at the above-described temperature for 10 minutes to 5 hours in the step (S1).

In the present invention, the method further includes allowing the metal oxide nanocrystals to grow after the step (S2). In the present invention, nanocrystals prior to growth may be represented as nanoseeds.

Metal oxide nanoseeds have limitations in industrial use because they have a small size and low optical properties. Therefore, in order to adjust the size of the nanoseeds to further enhance the optical properties or strengthen the catalytic effect of nanocrystals, the metal oxide nanoseeds bound to surfaces of the iron oxide nanoclusters may be used as seeds in the present invention to grow crystals around the seeds.

According to one embodiment, the nanoseeds may be spherical, and the particle size of the spherical nanoseeds may increase or grow into a spike shape or acicular shape in this step.

According to one embodiment, this step may be performed by mixing the multi-component mesocrystalline nanoparticles manufactured in the previous step with a metal ion precursor solution, an adjuvant, and an additive. The above-described types of metal ion precursors may be used as the metal ion precursor. The adjuvant serves to assist the growth of crystals, and hexamethylenetetramine (C₆H₁₂N₄, HMTA) may be used as the adjuvant. Also, the additive serves to assist the vertical growth of crystals, and ammonium chloride (NH₄Cl) and polyethylenimine (H(NHCH₂CH₂)nNH₂, PEI) may be used as the additive.

According to one embodiment, the growth of the metal oxide nanoseeds may be regulated by varying the content of the metal ion precursor.

Also, the present invention relates to a composition for a catalyst, a composition for hyperthermia therapy, a composition for image diagnosis, a kit for detecting an analyte, a molecular diagnostic chip, or a composition for delivery of a drug, which includes the multi-component mesocrystalline nanoparticles as described above. In this case, the catalyst may be a photocatalyst.

The multi-component mesocrystalline nanoparticles according to the present invention may be used to concentrate disease markers such as nucleic acids and/or proteins, and may be used as a reagent for a high-sensitivity diagnostic device. Also, the multi-component mesocrystalline nanoparticles may be used in a rapid kit for point-of-care testing (POCT) and may be used as scanner materials for diagnostic tests. Also, the multi-component mesocrystalline nanoparticles of the present invention may be used as a catalyst material, a nanoink material, a water treatment material, and an anticancer drug delivery system.

Also, the present invention relates to a method of purifying contaminated water, which includes:
allowing the multi-component mesocrystalline nanoparticles of the present invention and contaminated water to react under ultraviolet or visible light to decompose contaminants in the wastewater; and
recovering the multi-component mesocrystalline nanoparticles using a magnet.

The multi-component mesocrystalline nanoparticles according to the present invention absorb light to form photoelectron-hole pairs, and photocharges are rapidly separated through nanocrystalline junctions and is subjected to a photocatalytic reaction to form hydroxyl radicals. Also, the electrons reduce iron oxide (Fe³⁺) ions into Fe²⁺ and cause a photo-Fenton catalytic reaction to form hydroxyl radicals, thereby completely removing toxic substances present in the contaminated water (wastewater).

In the present invention, the contaminated water may include, for example, chlorinated compounds including a chlorophenol, such as 2,4,6-trichlorophenol, pentachlorophenol, and the like; or environmentally harmful substances having an aromatic ring, such as phenols, dioxine-based environmental hormones, dyes, or the like.

The multi-component mesocrystalline nanoparticles according to the present invention may be easily recovered using a magnet. The recovered multi-component mesocrystalline nanoparticles are well dispersed in water and readily recycled, thereby removing harmful environmental pollutants at low cost.

Further, the present invention relates to a method of detecting or imaging an analyte, which includes:
functionalizing biomolecules capable of binding to an analyte to be detected on surfaces of magnetic-optical composite nanostructures;
functionalizing biomolecules capable of binding to an analyte to be detected on surfaces of the multi-component mesocrystalline nanoparticles;
exposing the functionalized multi-component mesocrystalline nanoparticles to a sample including one or more analytes; and
identifying the analytes bound to the multi-component mesocrystalline nanoparticles.

The multi-component mesocrystalline nanoparticles according to the present invention may include the functionalized biomolecules capable of recognizing the analyte to be detected, and thus may be used as a probe applicable to detection of various biomolecules.

According to one embodiment, the analyte to be detected may be an amino acid, a peptide, a polypeptide, a protein, a glycoprotein, a lipoprotein, a nucleoside, a nucleotide, an oligonucleotide, a nucleic acid, a sugar, a carbohydrate, an oligosaccaride, a polysaccaride, a fatty acid, a lipid, a hormone, a metabolite, a cytokine, a chemokine, a receptor, a neurotransmitter, an antigen, an allergen, an antibody, a matrix, a metabolite, a cofactor, an inhibitor, a drug, a nutrient, a prion, a toxin, an explosive, a pesticide, a chemical weapon agent, a biohazard agent, a radioactive isotope, a vitamin, a heterocyclic aromatic compound, a carcinogen, a mutagen, an anesthetic, an amphetamine, a barbiturate, a hallucinogenic drug, a waste product, or a contaminant. Also, when the analyte is a nucleic acid, the nucleic acid may be a gene, viral RNA and DNA, bacterial DNA, fungal DNA, mammalian DNA, cDNA, mRNA, RNA and DNA fragments, an oligonucleotide, a synthetic oligonucleotide, a modified oligonucleotide, single- and double-stranded nucleic acids, natural and synthetic nucleic acids.

According to one embodiment, the biomolecules capable of binding to the surfaces of the multi-component mesocrystalline nanoparticles of the present invention that may recognize the analyte may be an antibody, an antibody fragment, a genetically engineered antibody, a single-chain antibody, a receptor protein, a binding protein, an enzyme, an inhibitor protein, a lectin, a cell adhesion protein, an oligonucleotide, a polynucleotide, a nucleic acid, or an aptamer.

Hereinafter, the present invention will be described in further detail with reference to examples thereof. However, it will be apparent to those skilled in the art that the examples described below are merely provided for exemplary illustration of the present invention, and are not intended to limit the scope of the present invention.

### EXAMPLES

### Example 1-1: Synthesis of iron oxide-zinc ferrite-zinc oxide multi-component mesocrystalline nanoparticles using continuous process (1-step process)

Synthesis of iron oxide-zinc ferrite-zinc oxide multi-component mesocrystalline nanoparticles was performed using a polyol method in one reaction system.

Iron chloride hexahydrate (FeCl₃·6H₂O) was used as an iron ion precursor, and ethylene glycol was used as a reducing agent and a solvent. Also, sodium acetate and sodium acrylate were used as anionic ligands, and distilled water (H₂O) was used as an adjuvant for assisting hydrolysis.

The above materials (0.54 g of iron chloride hexahydrate (FeCl3·6H₂O), 50 mL of ethylene glycol, 1.23 g of sodium acetate, 0.05 g of sodium acrylate, and 2.70 g of distilled water) were mixed, and put into a 3-neck flask. Thereafter, the resulting mixture was heated to a high temperature (200°C). After the elapse of a predetermined period of time, a solution obtained by dissolving 0.05 g of zinc acetate dihydrate (Zn(CH₃COO)₂·2H₂O) in 10 mL of ethylene glycol was rapidly injected into a reaction system, and then heated to 200°C. Then, the reaction solution was cooled to room temperature, and an alcohol was added thereto to wash the reaction solution using a centrifuge.

### Comparative Example 1-1: Synthesis of iron oxide-zinc oxide multi-component nanoparticles using 2-step polyol method

Iron oxide nanoparticles were first synthesized, and multi-component nanoparticles were then synthesized using a method of forming zinc oxide nanoparticles.

In a synthesis reaction of the iron oxide nanoparticles, iron chloride hexahydrate (FeCl₃·6H₂O) was used as the iron ion precursor, and ethylene glycol was used as the reducing agent and the solvent. Also, sodium acetate was used as the anionic ligand, and distilled water (H₂O) was used as the adjuvant for assisting hydrolysis.

The above materials (0.54 g of iron chloride hexahydrate (FeCl_{3·}6H₂O), 50 mL of ethylene glycol, 1.23 g of sodium acetate, and 2.70 g of distilled water) were mixed, and put into a 3-neck flask. Thereafter, the resulting mixture was heated to a high temperature (200°C). Then, the reaction solution was cooled to room temperature, and ethanol was added thereto to wash the reaction solution using a centrifuge (synthesis of iron oxide nanoparticles).

The synthesized iron oxide nanoparticles were dispersed again in 30 mL of diethylene glycol having a higher reducing power than ethylene glycol, and 0.05 g of zinc acetate dihydrate was added thereto. Thereafter, the resulting mixture was heated to a high temperature (200°C). Then, the reaction solution was cooled to room temperature, and ethanol was added thereto to wash the reaction solution using a centrifuge. Subsequently, the reaction solution was purified again using a magnet to manufacture the iron oxide-zinc oxide multi-component nanoparticles.

### Comparative Example 2-1

Multi-component nanoparticles were manufactured in the same manner as in Example 1, except that sodium acrylate was not used as the additive during the synthesis of the multi-component nanoparticles.

### Comparative Example 3-1

Multi-component nanoparticles were manufactured in the same manner as in Example 1, except that the polymer (poly(acrylic acid) sodium salt (PAANA)) was used instead of the monomer (sodium acrylate) during the synthesis of the multi-component nanoparticles.

### Example 1-2: Synthesis of spike-shaped particles

The multi-component mesocrystalline nanoparticles manufactured in Example 1-1 was dispersed in an aqueous solution, or fixed on a substrate and then immersed in an aqueous solution. Thereafter, the resulting dispersion was subjected to a hydrothermal synthesis method to synthesize spike-shaped particles. Specifically, the zinc oxide nanoparticles positioned outside the multi-component mesocrystalline nanoparticles synthesized in Example 1-1 were used as seeds to grow zinc oxide nanowires.

In this case, zinc nitrate hexahydrate (Zn(NO₃)₂·6H₂O) and hexamethylenetetramine (C₆H₁₂N₄, HMTA) were used as a zinc ion precursor and an adjuvant for assisting the synthesis of nanowires, respectively, and ammonium chloride (NH₄Cl) and polyethylenimine (H(NHCH₂CH₂)nNH₂, PEI) were used as additives for assisting the vertical growth of nanowires. The shape of the nanowires growing on the surfaces was able to be regulated by adjusting amounts of the zinc ion precursor, the adjuvant, and the additive.

The above materials (0.15 g of zinc nitrate hexahydrate, 0.07 g of hexamethylenetetramine, 0.16 g of ammonium chloride, 0.08 g of polyethylenimine, and 100 mL of water) were mixed, put into a hydrothermal synthesis system, and then heated to 94°C for 2 hours. The reaction solution was physically stirred during the reaction. Then, the reaction solution was cooled to room temperature, and water was then added to magnetically separate spike-shaped nanoparticles. Then, ethanol was added thereto to wash the reaction solution using a centrifuge.

### Example 2-1: Synthesis of iron oxide-cerium ferrite-cerium oxide multi-component mesocrystalline nanoparticles using continuous process (1-step process)

Iron oxide-cerium ferrite-cerium oxide multi-component mesocrystalline nanoparticles were manufactured in the same manner as in Example 1-1, except that cerium acetate (Ce(CH₃COO)·xH₂O) was used as the metal ion precursor during the synthesis of the multi-component mesocrystalline nanoparticles.

### Example 3-1: Synthesis of iron oxide-manganese ferrite-manganese oxide multi-component mesocrystalline nanoparticles using continuous process (1-step process)

Iron oxide-manganese ferrite-manganese oxide multi-component mesocrystalline nanoparticles were manufactured in the same manner as in Example 1-1, except that manganese acetate tetrahydrate (Mn(CH₃COO)₂·2H₂O) was used as the metal ion precursor during the synthesis of the multi-component mesocrystalline nanoparticles.

### Experimental Example 1: Physical properties of multi-component mesocrystalline nanoparticles

In the present invention, FIG. 1 shows a schematic diagram of a method of manufacturing multi-component mesocrystalline nanoparticles using the polyol method of Example 1-1, which is a continuous process.

FIG. 2 shows transmission electron microscope images and energy dispersive spectrometry (EDS) images of the multi-component mesocrystalline nanoparticles manufactured by the polyol method (continuous process) in Example 1-1.

As shown in FIG. 2, it can be seen that the multi-component mesocrystalline nanoparticles were composed of iron oxide, zinc ferrite, and zinc oxide nanocrystals. In particular, it can be seen that the multi-component mesocrystalline nanoparticles had a structure in which zinc ferrite and zinc oxide nanoparticles were bound to surfaces of the iron oxide nanoparticles of the iron oxide nanoclusters.

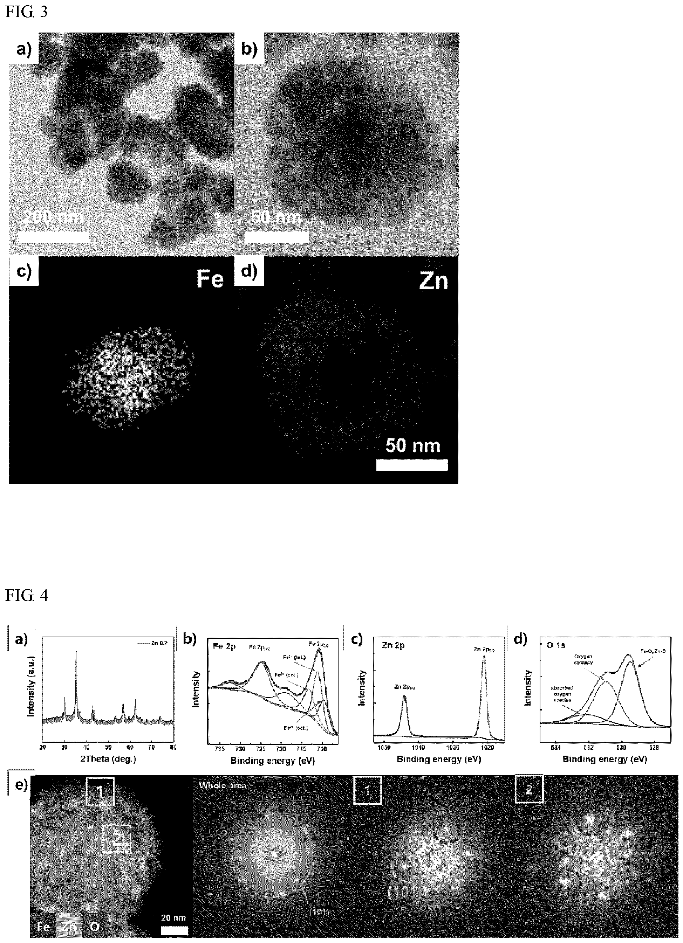

Meanwhile, FIG. 3 shows transmission electron microscope images (a and b) and energy dispersive spectrometry images (c and d) of the multi-component nanoparticles manufactured by the 2-step polyol method in Comparative Example 1-1.

As shown in FIG. 3, it can be seen that a zinc oxide shell was formed on the iron oxide nanoclusters. However, it can be seen that many particles were aggregated because it was difficult to adjust the zinc oxide layers present on the surfaces to be uniformly distributed on the nanoparticles, compared to the multi-component mesocrystalline nanoparticles manufactured in Example 1-1. Therefore, the nanoparticles had a problem in that the nanoparticles were not easily dispersed in an aqueous solution. Above all, the nanoparticles had a drawback in that a synthesis process took a lot of time.

FIG. 4 shows the X-ray diffraction pattern results and selected area electron diffraction (SAED) pattern images of the multi-component mesocrystalline nanoparticles manufactured in Example 1-1.

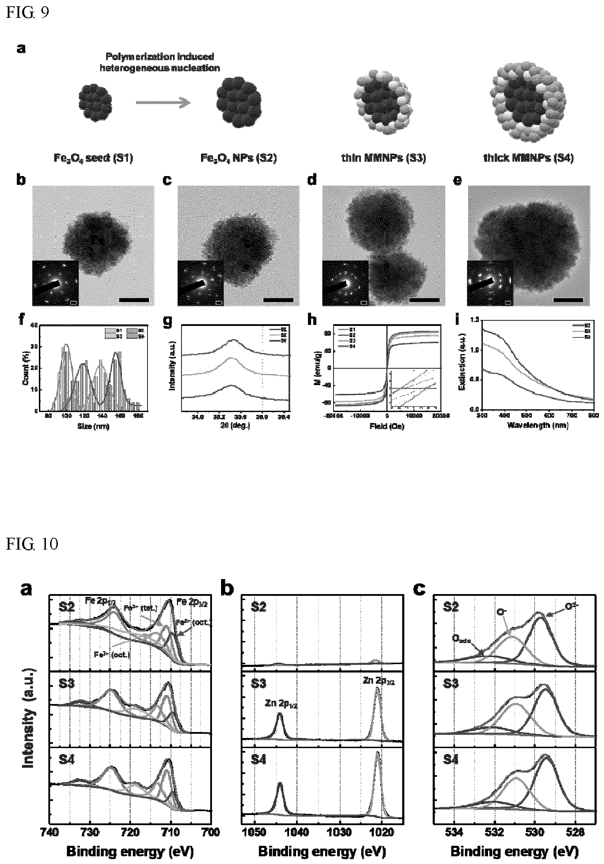

As shown in FIG. 4, it can be seen that the X-ray diffraction pattern of the zinc oxide was not observed, but the zinc oxide had a short-range order, as determined by XPS and HR-TEM analyses, and the like. Also, it can be seen that the zinc ferrite (ZnFe₂O₄) was somewhat formed as the X-ray diffraction pattern shifted slightly toward the left. Meanwhile, an enlarged view of the X-ray diffraction pattern is shown in FIG. 9g.

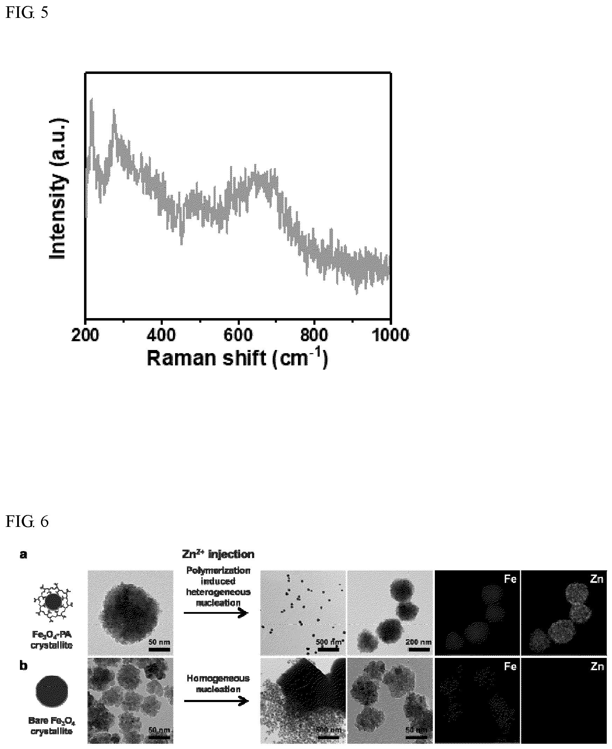

FIG. 5 shows the results of measuring the Raman spectrum of the multi-component mesocrystalline nanoparticles manufactured in Example 1-1 using a Raman spectrometer.

As shown in FIG. 5, it can be seen that Raman peaks arising from zinc ferrite are observed at 213, 278, 490, and 639 cm⁻¹, which are clearly distinguished from the iron oxide whose peak is observed at 680 cm⁻¹, indicating that the zinc ferrite was present.

### Experimental Example 2: Comparison of characteristics of multi-component mesocrystalline nanoparticles according to presence and absence of acrylate

The iron oxide-zinc ferrite-zinc oxide multi-component mesocrystalline nanoparticles manufactured in Example 1-1 and the iron oxide-zinc oxide multi-component nanoparticles manufactured in Example 2-1 were compared.

In the present invention, FIG. 6 shows transmission electron microscope images and energy dispersive spectrometry images of the iron oxide clusters and the multi-component mesocrystalline nanoparticles according to the presence (i.e., Example 1-1) and absence (i.e., Comparative Example 2-1) of polyacrylate.

Specifically, FIG. 6a shows an image of the iron oxide-zinc oxide multi-component nanoparticles manufactured in Comparative Example 2-1, and FIG. 6b shows an image of the iron oxide-zinc ferrite-zinc oxide multi-component mesocrystalline nanoparticles manufactured in Example 1-1.

As shown in FIG. 6, it can be seen from the energy dispersive spectrometry image that the zinc oxide particles were formed on surfaces of the multi-component mesocrystalline nanoparticles according to Example 1-1 of the present invention in which the Na acrylate was added, but the zinc oxide crystals were separately formed in the case of Comparative Example in which the Na acrylate was not added.

Also, the surface state of the multi-component mesocrystalline nanoparticles at the time point of injection of the zinc ion precursor according to the addition of sodium acrylate was analyzed in this example.

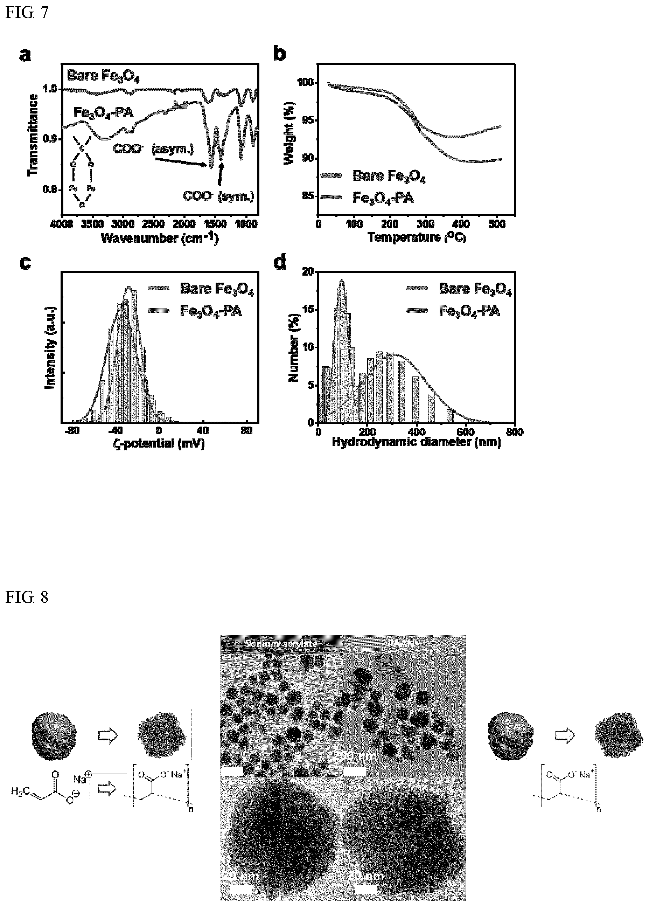

The results are shown in FIG. 7. Specifically, the Fourier transform infrared spectrum, thermogravimetric analysis, zeta potential spectrum, and hydrodynamic size distribution results are shown. In this case, the expression "Bare Fe₃O₄" represents the results of Comparative Example 2-1, and the expression "Fe₃O₄-PA" represents the results of Example 1-1.

As shown in FIG. 7, it can be seen that the surfaces of the iron oxide nanoparticles were coated with the polyacrylate at the time point of injection of the zinc ion precursor in the case of Examples. Because the zinc ferrite and the zinc oxide were bound through the acrylate, the multi-component mesocrystalline nanoparticles according to the present invention could be easily manufactured.

Also, it can be seen that the surface zeta potential was stronger, and the hydrodynamic size distribution was also narrower at a smaller size.

### Experimental Example 3: Comparison of characteristics of multi-component mesocrystalline nanoparticles according to use of acrylate or polyacrylate

The iron oxide-zinc ferrite-zinc oxide multi-component mesocrystalline nanoparticles manufactured in Example 1-1 and the multi-component nanoparticles manufactured in Comparative Example 3-1 were compared.

In the present invention, FIG. 8 shows transmission electron microscope images of the iron oxide-zinc ferrite-zinc oxide multi-component mesocrystalline nanoparticles. In this case, the expression "Sodium acrylate" represents the iron oxide-zinc ferrite-zinc oxide multi-component mesocrystalline nanoparticles manufactured in Example 1-1, and the expression "PAANa" represents the multi-component nanoparticles manufactured in Comparative Example 3-1, that is, manufactured using the polymer (e. g., PAANa).

As shown in FIG. 8, it can be seen that the byproducts were also generated when the polymer (e. g., PAANa) was used. Also, it can be seen that, when the composition was analyzed on the surfaces of single multi-functional mesocrystalline nanoparticles, the nanoparticles manufactured in Example 1-1 had an Fe:Zn injection ratio of 10:1, whereas the nanoparticles manufactured in Comparative Example 3-1 had an Fe:Zn injection ratio of 15:1, indicating that the ZnFe₂O₄ and ZnO nanocrystals are more sparsely formed in the nanoparticles manufactured in Comparative Example 3-1.

### Experimental Example 4: Comparison of characteristics of multi-component mesocrystalline nanoparticles according to content of zinc ion precursor

The characteristics of the iron oxide-zinc ferrite-zinc oxide multi-component mesocrystalline nanoparticles according to the content of the zinc ion precursor were compared.

FIG. 9a schematically shows the multi-component mesocrystalline nanoparticles formed by injection of the precursor. FIGS. 9b to 9e are transmission electron microscope images of an iron oxide cluster before injection of the zinc ion precursor (b), an iron oxide cluster which was reacted for the same time as in 9d and 9e without addition of the zinc ion precursor (c), multi-component mesocrystalline nanoparticles into which Fe and Zn were injected at an injection ratio of 10:1 (d), and multi-component mesocrystalline nanoparticles into which Fe and Zn were injected at an injection ratio of 10:2 (e). In FIGS. 9f, 9g, 9h and 9i, the iron oxide clusters and the multi-component mesocrystalline nanoparticles of (b) to (e) were designated as S1, S2, S3, and S4, respectively.

As shown in FIG. 9f, it can be seen that the size of the multi-component mesocrystalline nanoparticles increased with an increasing content of the zinc ion precursor.

As shown in FIG. 9h, it can be seen that the saturation magnetization intensity of the iron oxide clusters increased with an increasing reaction time. Also, it can be seen that the formation of the zinc ferrite and zinc oxide nanocrystal reduced the saturation magnetization intensity and lowered magnetic susceptibility and a coercive force.

As shown in FIG. 9i, it can also be seen that the absorbance of visible light increased with an increasing amount of the metal oxide nanocrystals present on the iron oxide clusters.

Also, in the present invention, FIG. 10 shows the X-ray photoelectron spectroscopy results of the iron oxide-zinc ferrite-zinc oxide multi-component mesocrystalline nanoparticles according to the content of the zinc ion precursor. FIG. 10a shows the Fe 2p spectra of S2, S3, and S4, FIG. 10b shows the Zn 2p spectra of S2, S3, and S4, and FIG. 10c shows the O Is spectra of S2, S3, and S4.

As shown in FIG. 10, it can be seen that some of the Fe²⁺ was replaced with Zn²⁺ because the signals originating from Fe²⁺ decreased as the zinc ion precursor was injected. Also, it can be seen from FIG. 10c that an excessive amount of Zn²⁺ was present in the form of zinc oxide because a larger amount of Zn²⁺ was present relative to the corresponding increasing/decreasing amount of the signals.

### Experimental Example 5: Physical properties of multi-component mesocrystalline nanoparticles

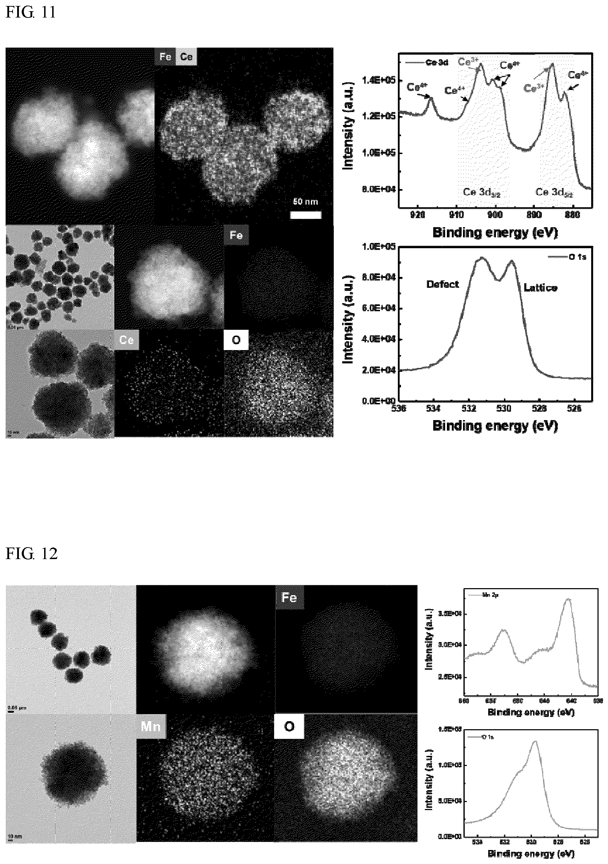

In the present invention, FIG. 11 shows transmission electron microscope (TEM) images, energy dispersive spectrometry (EDS) images, and the X-ray photoelectron spectroscopy results of the multi-component mesocrystalline nanoparticles manufactured by the manufacturing method in Example 2-1.

As shown in FIG. 11, it can be seen that the multi-component mesocrystalline nanoparticles had the same heterogeneous nanocrystalline structure as the multi-component mesocrystalline nanoparticles manufactured in Example 1-1.

In the present invention, FIG. 12 shows transmission electron microscope (TEM) images, energy dispersive spectrometry (EDS) images, and the X-ray photoelectron spectroscopy results of the multi-component mesocrystalline nanoparticles manufactured by the manufacturing method in Example 3-1.

As shown in FIG. 12, it can be seen that the multi-component mesocrystalline nanoparticles had the same heterogeneous nanocrystalline structure as the multi-component mesocrystalline nanoparticles manufactured in Example 1-1.

### Experimental Example 6: Confirmation of photocatalytic properties using iron oxide-zinc ferrite-zinc oxide multi-component mesocrystalline nanoparticles

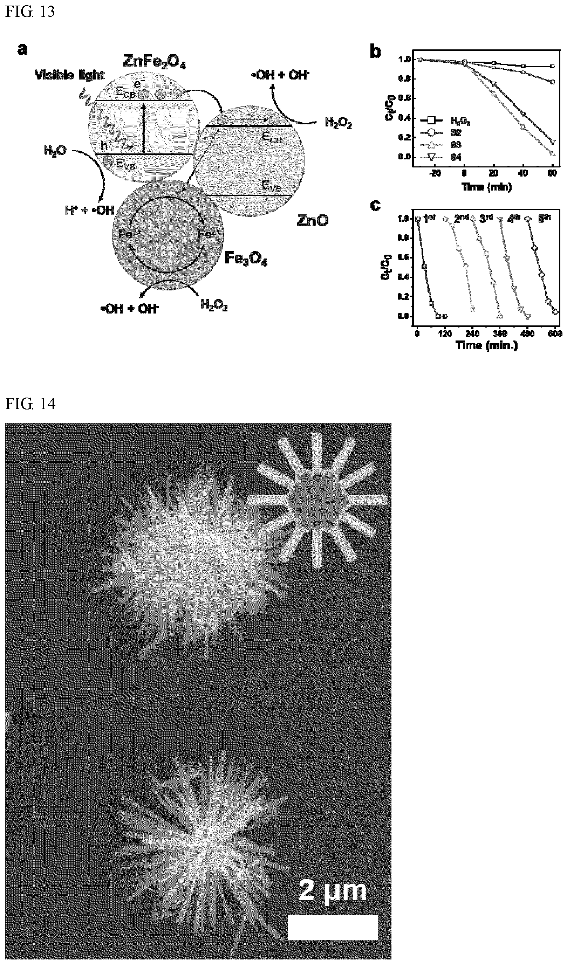

As shown in FIG. 13 of the present invention, a large number of junctions formed between unit crystals are formed in the iron oxide-zinc ferrite-zinc oxide multi-component mesocrystalline nanoparticles. Zinc ferrite nanocrystals having a band gap of 2 eV absorb visible light to form photoelectron-hole pairs. Photocharges are rapidly separated through nanocrystalline junctions so that the holes are allowed to react with water molecules on a surface of zinc ferrite, thereby forming hydroxyl radicals through a photocatalytic reaction. On the other hand, the electrons are transferred to the zinc oxide nanocrystals. In this case, some electrons react with hydrogen peroxide to form hydroxyl radicals, and the other electrons are transferred to iron oxide to reduce Fe³⁺ions into Fe²⁺. As a result, a photo-Fenton catalytic reaction is triggered to generate hydroxyl radicals.

When the multi-functional mesocrystalline nanoparticles were added to a contaminated aqueous solution containing organic pollutants (model molecule: methylene blue) and irradiated with visible light, hydroxyl radicals were formed to decompose all the organic pollutant in one hour. After the purification of contaminated water, it was confirmed that the multi-functional mesocrystalline nanoparticles used were recovered using a permanent magnet, and the catalytic function of the multi-functional mesocrystalline nanoparticles was maintained while performing a recycling test 5 times.

### Experimental Example 7: Physical properties of spike-shaped iron oxide-zinc ferrite-zinc oxide multi-component nanoparticles

FIG. 14 shows a schematic diagram and a scanning electron microscope image of the iron oxide-zinc ferrite-zinc oxide multi-component nanoparticles with a spike shape synthesized by a hydrothermal synthesis method.

As shown in FIG. 14, it can be seen that the iron oxide- zinc ferrite-zinc oxide multi-component nanoparticles with a spike shape had a length of approximately 600 nm and a thickness of 50 nm.

According to the present invention, the multi-component mesocrystalline nanoparticles, which consist of different types of metal oxides and have other properties such as catalytic (specifically, photocatalytic), light emission, and antioxidant actions as well as magnetic properties, can be manufactured using a simple and stable method. Also, the contents of the different metal ion precursors and the content ratio of the precursor to the solvent can be adjusted to adjust the size of the iron oxide nanoclusters and the size of the building blocks (e.g., metal oxide nanocrystals). Through this, the unique characteristics, the size characteristics, and the aggregate characteristics of a material can be adjusted.

Also, the surfaces of the multi-component mesocrystalline nanoparticles manufactured according to the present invention can be freely modified to enhance dispersibility in a solvent and perform bio-functionalization and complexation with other nanostructures.

While the invention has been shown and described with reference to certain exemplary embodiments thereof, it will be understood by those skilled in the art that various changes in form and details may be made therein without departing from the spirit and scope of the invention as defined by the appended claims.

## Claims

1. A multi-component mesocrystalline nanoparticle comprising:
an iron oxide nanocluster; and
metal oxide nanocrystals bound to an iron oxide surface of the iron oxide nanocluster,
wherein the metal oxide nanocrystals are bound to acrylate groups formed on the iron oxide surface.

2. The multi-component mesocrystalline nanoparticle of claim 1, wherein an iron oxide in the iron oxide nanocluster comprises one or more selected from the group consisting of Fe₂O₃, Fe₃O₄, CoFe₂O₄, NiFe₂O₄, ZnFe₂O₄, MgFe₂O₄, and MnFe₂O₄.

3. The multi-component mesocrystalline nanoparticle of claim 1, wherein a metal oxide in the metal oxide nanocrystals comprises one or more selected from the group consisting of zinc oxide (ZnO), cerium oxide (CeO₂), manganese oxide (MnO₂), nickel oxide (NiO, Ni₂O₃), cobalt oxide (Co₃O₄, CoO), magnesium oxide (MgO), zinc ferrite (ZnFe₂O₄), cerium ferrite (CeFe₂O₄), manganese ferrite (MnFe₂O₄), nickel ferrite (NiFe₂O₄), cobalt ferrite (CoFe₂O₄), magnesium ferrite (MgFe₂O₄), and cerium-doped iron oxide (CeₓFe₃₋ₓO₄).

4. The multi-component mesocrystalline nanoparticle of claim 1, wherein the iron oxide nanocluster has an average particle size of 10 to 500 nm, and
the metal oxide nanocrystals have an average particle size of 1 to 100 nm.

5. The multi-component mesocrystalline nanoparticle of claim 1, wherein the metal oxide nanocrystals are spherical, spike-shaped, or acicular.

6. A method of manufacturing multi-component mesocrystalline nanoparticles defined in claim 1, the method comprising:
allowing a mixture comprising an iron ion precursor, an anionic ligand, and a solvent to react at 100 to 300°C; and
injecting a metal ion precursor solution into the reaction product of the previous step to react with the reaction product,
wherein the anionic ligand comprises an acetate-based compound and an acrylate-based compound.

7. The method of claim 6, which is performed in a single-stage reactor.

8. The method of claim 6, wherein the iron ion precursor is iron chloride hexahydrate (FeCl₃·6H₂O).

9. The method of claim 8, wherein the mixture further comprises one or more selected from the group consisting of zinc chloride (ZnCl₂), magnesium chloride hexahydrate (MgCl₂·6H₂O), manganese chloride tetrahydrate (MnCl₂·4H₂O), nickel chloride hexahydrate (NiCl₂·6H₂O), and cobalt chloride hexahydrate (CoCl₂·6H₂O).

10. The method of claim 6, wherein the acetate-based compound comprises one or more selected from the group consisting of sodium acetate, potassium acetate, and ammonium acetate, and
the acrylate-based compound comprises one or more selected from the group consisting of sodium acrylate, poly(acrylic acid), and poly(acrylic acid sodium salt).

11. The method of claim 6, wherein the acetate-based compound and the acrylate-based compound are included at a ratio of 10000:1 to 1:1.

12. The method of claim 6, wherein the solvent is ethylene glycol, diethylene glycol, triethylene glycol, or tetraethylene glycol.

13. The method of claim 6, wherein a metal ion precursor in the metal ion precursor solution comprises one or more selected from the group consisting of zinc acetate dihydrate (Zn(CH₃COO)₂·2H₂O), manganese acetate dihydrate (Mn(CH₃COO)₂·2H₂O), cerium acetate (Ce(CH₃COO)₃·xH₂O), magnesium acetate tetrahydrate (Mg(CH₃COO)₂·4H₂O), cobalt acetate tetrahydrate (Co(CH₃COO)₂·4H₂O), and nickel acetate tetrahydrate (Ni(CH₃COO)₂·4H₂O).

14. The method of claim 6, wherein a content of the metal ion precursor is in a range of 0.01 mmol to 1 mol.

15. The method of claim 6, further comprising:
allowing the metal oxide nanocrystals to grow on the manufactured multi-component mesocrystalline nanoparticles.

16. A composition for a catalyst, a composition for hyperthermia therapy, a composition for image diagnosis, a kit for detecting an analyte, a molecular diagnostic chip, or a composition for delivery of a drug, which comprises the multi-component mesocrystalline nanoparticles defined in claim 1.

17. A method of purifying contaminated water, the method comprising:
allowing the multi-component mesocrystalline nanoparticles defined in claim 1 and contaminated water to react under ultraviolet or visible light to decompose contaminants in the wastewater; and
recovering the multi-component mesocrystalline nanoparticles using a magnet.

18. A method of detecting or imaging an analyte, the method comprising:
functionalizing biomolecules capable of binding to an analyte to be detected on surfaces of the multi-component mesocrystalline nanoparticles defined in claim 1;
exposing the functionalized multi-component mesocrystalline nanoparticles to a sample comprising one or more analytes; and
identifying the analytes bound to the multi-component mesocrystalline nanoparticles.
